Europäisches Patentamt

European Patent Office

Office européen des brevets

(19)

(11) Veröffentlichungsnummer: **0 221 325**
**B1**

(12) **EUROPÄISCHE PATENTSCHRIFT**

(45) Veröffentlichungstag der Patentschrift:
**25.07.90**

(51) Int. Cl.[5]: **A61B 17/22**

(21) Anmeldenummer: **86113196.9**

(22) Anmeldetag: **25.09.86**

(54) **Lithotripsie-Einheit.**

(30) Priorität: **09.10.85 DE 3536091**
**06.12.85 DE 3543265**

(43) Veröffentlichungstag der Anmeldung:
**13.05.87 Patentblatt 87/20**

(45) Bekanntmachung des Hinweises auf die Patenterteilung:
**25.07.90 Patentblatt 90/30**

(84) Benannte Vertragsstaaten:
**DE FR GB IT**

(56) Entgegenhaltungen:
**EP-A- 0 133 946**
**EP-A- 0 167 670**
**EP-A- 0 205 878**
**WO-A-85/03631**
**DE-A- 3 220 751**
**GB-A- 2 038 150**

(73) Patentinhaber: **Siemens Aktiengesellschaft,**
**Wittelsbacherplatz 2, D-8000 München 2(DE)**

(72) Erfinder: **Heumann, Reiner, Am Mühlgarten 17,**
**D-8521 Spardorf(DE)**
Erfinder: **Rattner, Manfred, Am Eichengarten 8,**
**D-8520 Buckenhof(DE)**
Erfinder: **Noske, Erich, Ruhsteinweg 12,**
**D-8525 Weiher(DE)**

ACTORUM AG

**Beschreibung**

Eine Lithotripsie-Einheit wird in der Medizin eingesetzt, z.B. zum Zerstören von Nierensteinen. Es ist dabei bekannt, den Patienten in einer mit Wasser gefüllten Wanne zu lagern, über das Stoßwellen eines Stoßwellenerzeugers auf die Steine zu deren Zertrümmerung übertragen werden.

Aus der EP-A-0 167 670 ist es bekannt, eine Sendeantenne für auf einen zu zertrümmernden Stein fokussierte elektromagnetische Wellen unterhalb eines Patientenlagerungstisches anzuordnen.

Weiter ist es aus der DE-A-3 220 751 bekannt, bei einer Vorrichtung zur Zertrümmerung von im Körper eines Patienten befindlichen Konkrementen, insbesondere von Nierensteinen, eine Röntgenuntersuchungseinrichtung mit einer Röntgeneinheit vorzusehen, die den Patienten unter unterschiedlichen Winkeln durchstrahlt. Dabei schneiden sich die Zentralstrahlen der Röntgeneinheit in dem Fokus eines Stoßwellengenerators. Mit Hilfe der Ortungseinrichtung wird der Patient in Richtung der Achse des Stoßwellengenerators und senkrecht dazu so bewegt, daß der zu zerstörende Stein im Fokus der Stoßwellen liegt.

Eine Lithotripsie-Einheit mit einer in drei orthogonalen Richtungen verstellbaren Patientenliege, mit einem unterhalb der Patientenliege befindlichen Stoßwellengenerator, der über eine Membran an die Patientenoberfläche angekoppelt wird und derart verschiebbar ist, daß der Fokus der Stoßwellen mit einem Isozentrum zusammenfällt, und mit einer Röntgeneinrichtung, die den Patienten in unterschiedlichen Richtungen durchstrahlt, ist in der älteren, nicht vorveröffentlichten EP-A-0 205 878 beschrieben.

Der Erfindung liegt die Aufgabe zugrunde, eine universelle Lithotripsie-Einheit zu schaffen, die folgende Arbeiten ermöglicht:

1. Stoßwellenbehandlung, insbesondere Zertrümmerung von Nieren- und Uretersteinen.
2. Urologische Behandlungen einschließlich perkutaner Nephrostomie.
3. Röntgenuntersuchungen.

Diese Aufgabe ist erfindungsgemäß durch eine Lithotripsie-Einheit gelöst, die folgende Merkmale aufweist:

a) einen in drei Raumkoordinaten in bezug auf ein Isozentrum (9) verstellbaren Patientenlagerungstisch (1),

b) einen Stoßwellengenerator (3, 4) zur Zertrümmerung von im Körper eines Patienten befindlichen Steinen, wobei der Stoßwellengenerator (3, 4) über eine Membran an die Patientenoberfläche ankoppelbar ist und unter dem Patientenlagerungstisch (1) zumindest in Richtung seiner Achse (3a, 4a) derart einstellbar gelagert ist, daß eine Fokusverlagerung in das Isozentrum (9) möglich ist, und

c) eine Röntgenuntersuchungseinrichtung zur Ortung zu zertrümmender Steine, wobei die Röntgenuntersuchungseinrichtung eine Röntgeneinheit (5, 6; 7, 8) aufweist, mittels derer ein Patient unter unterschiedlichen Winkeln durchstrahlbar ist, wobei die durch die Zentralstrahlen (6a, 8a) der Röntgeneinheit (5, 6; 7, 8) definierte Ebene die Längsachse des Patientenlagerungstisches (1) enthält, derart, daß ein Zentralstrahl (6a) in anterior-posterior-Richtung und ein zweiter Zentralstrahl (8a) in caudal-cranialer Richtung verläuft und die Zentralstrahlen (6a, 8a) sich in dem Isozentrum (9) schneiden.

Eine besonders zweckmäßige Ausgestaltung der Erfindung besteht darin, daß beide Röntgenstrahler in einem gemeinsamen Gehäuse in einer vertikalen Ebene verstellbar gelagert sind. Bei dieser Ausgestaltung ist es möglich, die Röntgenstrahler im Untersuchungsraum dann, wenn sie nicht für die Ortung von Steinen benötigt werden, so weit nach oben zu verstellen, daß sie den Benutzer bei allen praktisch vorkommenden Tätigkeiten, insbesondere auch bei perkutanen Eingriffen, nicht behindern. Das Gehäuse kann dabei ein Universal-Anschlußstück zum wahlweisen Verbinden mit der Decke oder Wand des Untersuchungsraumes oder einer vertikalen Säule aufweisen.

Die Erfindung ist nachfolgend anhand eines in der Zeichnung dargestellten Ausführungsbeispieles näher erläutert. Es zeigen:

Fig. 1 und 2 zwei verschiedene Ansichten einer Lithotripsie-Einheit nach der Erfindung,

Fig. 3 und 4 den Ansichten gemäß Figuren 1 und 2 entsprechende Ansichten der Einheit mit geänderter Stellung eines Röntgenstrahlers,

Fig. 5 und 6 zwei verschiedene Ansichten einer anderen Ausbildung einer Lithotripsie-Einheit nach der Erfindung, und

Fig. 7 und 8 zwei verschiedene Ansichten einer Variante der Einheit gemäß den Figuren 5 und 6.

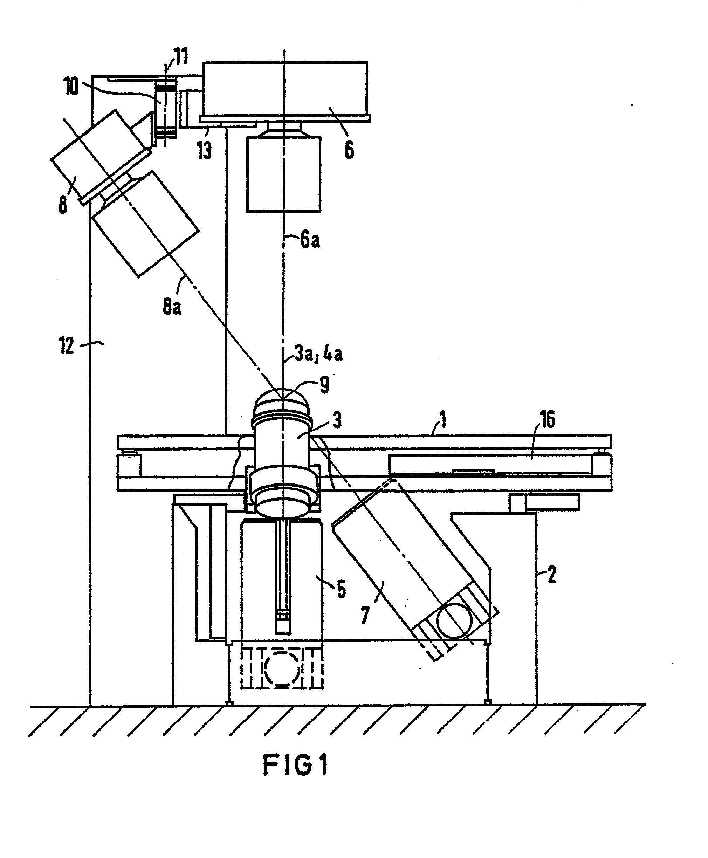

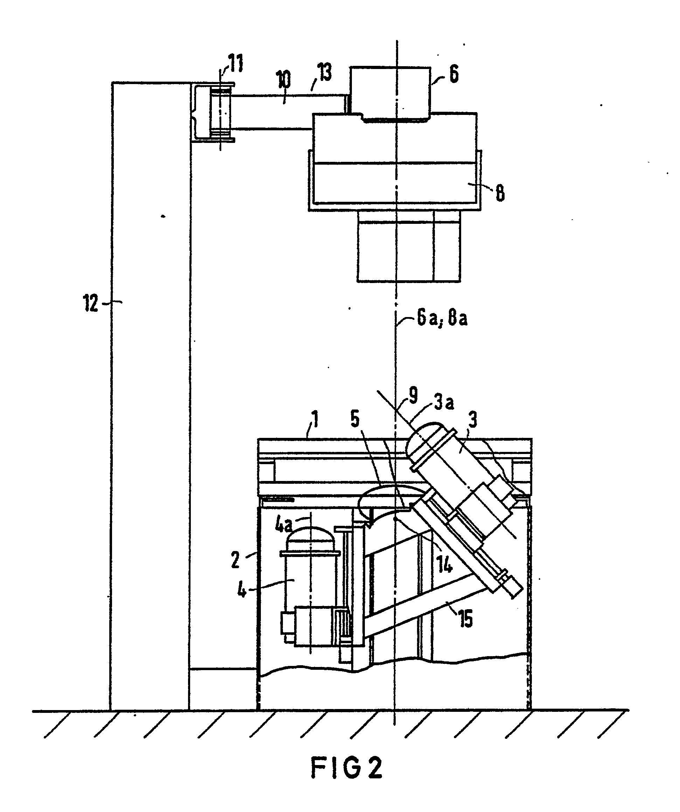

In den Figuren 1 und 2 ist ein in drei Raumkoordinaten verstellbarer Patientenlagerungstisch 1 mit einem Sockel 2 dargestellt, unter dem zwei Stoßwellengeneratoren 3, 4 angeordnet sind. In der Figur 1 ist der Stoßwellengenerator 4 durch einen Röntgenbildverstärker 5 verdeckt. Der Röntgenbildverstärker 5 bildet zusammen mit einem Röntgenstrahler 6 ein erstes Röntgensystem, bei dem der Röntgenstrahler 6 senkrecht zum Patientenlagerungstisch 1, d.h. in auterior-posterior-Richtung strahlt.

Ein zweites Röntgensystem ist von einem Röntgenbildverstärker 7 und einem Röntgenstrahler 8 gebildet. Der Röntgenstrahler 8 strahlt in caudal-cranialer Richtung.

Aufgrund der unterschiedlichen Richtungen der Zentralstrahlen 6a und 8a der Röntgensysteme 5, 6; 7, 8 ist eine Ortung des jeweils zu zertrümmernden Nierensteines möglich. Die Zentralstrahlen 6a, 8a schneiden sich dabei in einem Isozentrum 9, in das der zu zertrümmernde Nierenstein durch entsprechende Bewegung des Patientenlagerungstisches 1 verstellt wird.

Die durch die beiden Zentralstrahlen 6a, 8a definierte Ebene geht durch die Längsachse des Patientenlagerungstisches 1, so daß der seitliche Platzbedarf für die Röntgeneinheiten gering ist.

Der Röntgenstrahler 8 ist an einem Arm 10 befestigt, der um eine vertikale Achse 11 schwenkbar an einem Stativ 12 gelagert ist. Dadurch ist es möglich, den Röntgenstrahler 8 aus dem Arbeitsbereich herauszuschwenken.

Der Röntgenstrahler 6 ist ebenfalls mit dem Stativ 12 verbunden, und zwar mit Hilfe eines Armes 13, der es erlaubt, den Röntgenstrahler 6 quer zum Patientenlagerungstisch 1 zu verstellen. Auch der Röntgenstrahler 6 ist demgemäß aus dem Arbeitsbereich herausbewegbar.

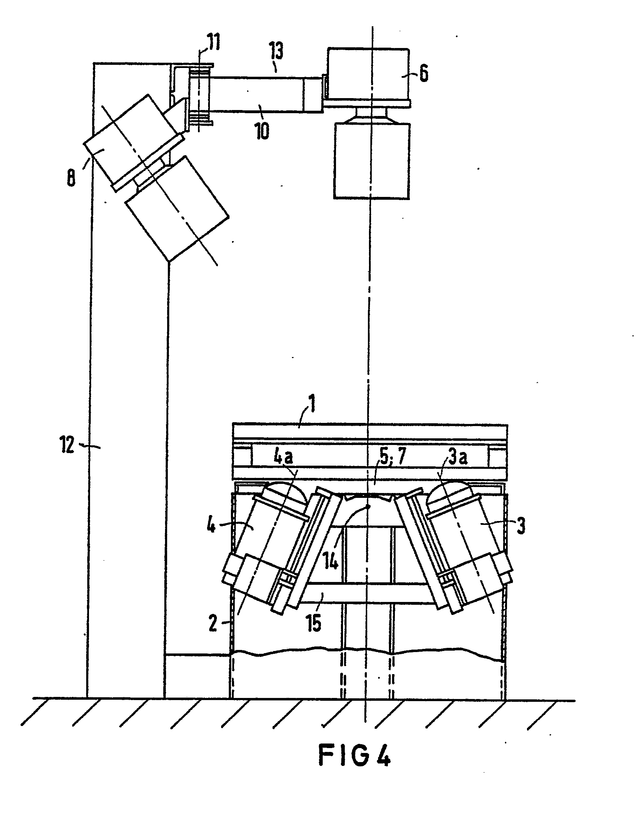

Die Stoßwellengeneratoren 3, 4 sind auf einem gemeinsamen, um eine parallel zur Tischlängsachse verlaufende Achse 14 schwenkbaren Halter 15 derart gelagert, daß sich ihre Achsen 3a, 4a unter einem Winkel schneiden (Figuren 2, 4) und jeder Stoßwellengenerator 3, 4 in Richtung seiner Achse 3a, 4a verstellbar ist.

Zur Stoßwellenbehandlung eines Patienten wird dieser auf dem Patientenlagerungstisch 1 gelagert und unter Röntgenkontrolle mit dem zu zertrümmernden Stein in das Isozentrum 9 bewegt. Anschließend wird einer der beiden Stoßwellengeneratoren 3, 4 mit seiner Achse 3a, 4a auf das Isozentrum ausgerichtet und an den Patienten durch eine Öffnung im Patientenlagerungstisch 1 herangefahren, bis er mit einer Membran an der Hautoberfläche des Patienten anliegt. Anschließend kann die Stoßwellenbehandlung erfolgen.

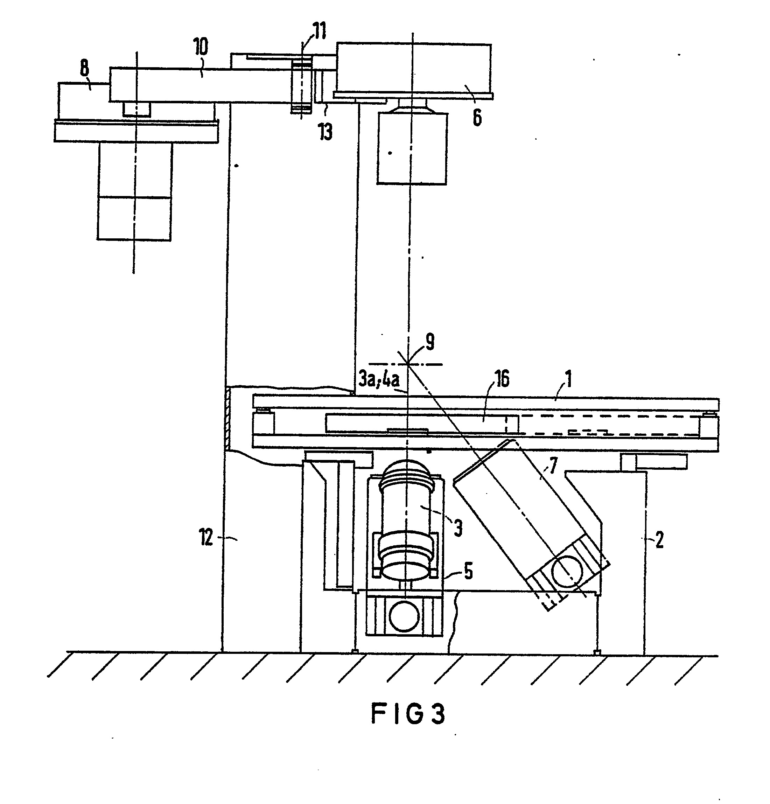

Für das Einbringen einer Röntgenfilmkassette können die Stoßwellengeneratoren 3, 4 mit Hilfe des Halters 15 in die in der Figur 4 gezeichnete Stellung gebracht werden, in der die Röntgenfilmkassette 16 in die in der Figur 3 gezeigte Arbeitsstellung verstellbar ist. In der Figur 1 nimmt die Röntgenfilmkassette 16 die Vorbereitungsstellung ein, in der der Raum unter dem zu zertrümmernden Stein für die Applikation eines der beiden Stoßwellengeneratoren 3, 4 freigelassen ist. Die Stoßwellengeneratoren 3, 4 sind dabei den beiden Nieren zugeordnet.

Der Patientenlagerungstisch 1 ist zum Anbau urologischer Hilfsgeräte, z.B. Beinhalter, ausgebildet, so daß auch übliche urologische Manipulationen durchgeführt werden können. Der dargestellte Lithotripsie-Arbeitsplatz kann auch als üblicher Röntgenarbeitsplatz für Durchleuchtung und Aufnahme benutzt werden.

Es ist auch möglich, auf die Schwenkung des Halters 15 zu verzichten, wenn dieser so ausgebildet wird, daß sich die Längsachsen der beiden Stoßwellengeneratoren 3, 4 im Isozentrum 9 schneiden.

Anstelle zweier Röntgensysteme zur Durchstrahlung des Patienten unter unterschiedlichen Winkeln kann auch nur ein solches System mit einem oder zwei Bildverstärkern vorhanden sein, wenn es entsprechend verstellbar gelagert ist. Auch ist es möglich, ein einziges ortsfestes Röntgensystem mit einem Bildverstärker vorzusehen, wenn eine Stereo-Röntgenröhre verwendet wird.

Wegen der als individuelle, an der Patientenoberfläche zu applizierende Einheiten ausgebildeten Stoßwellengeneratoren 3, 4 ist eine Ankopplung über eine wassergefüllte Wanne nicht erforderlich.

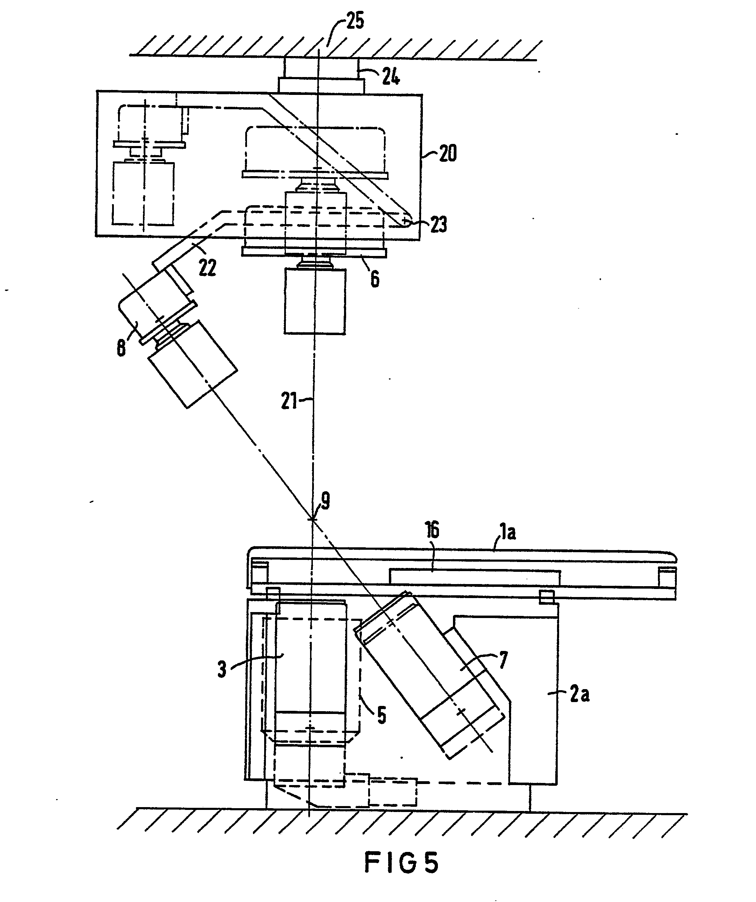

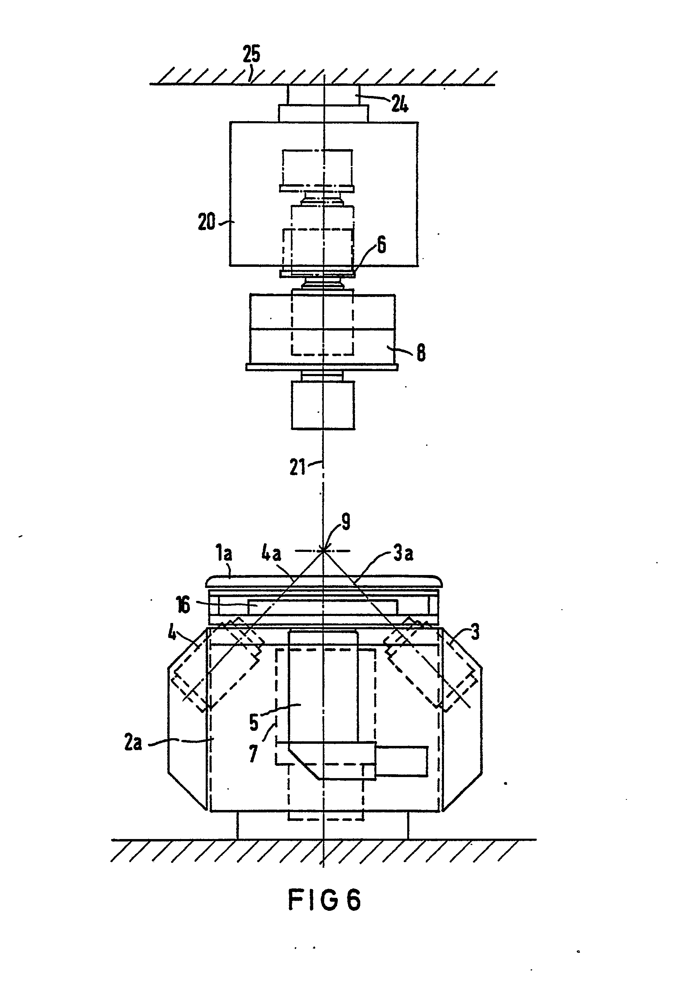

Bei der Ausführungsform der Erfindung gemäß den Figuren 5 und 6 sind Teile, die mit Teilen der Figuren 1 bis 4 gleich sind, mit den gleichen Bezugszeichen bezeichnet. Der Patientenlagerungstisch 1a ist dabei etwas kürzer als der Patientenlagerungstisch 1 ausgeführt. Die Stoßwellengeneratoren 3, 4 sind nur in Richtung der Achsen 3a, 4a verstellbar, welche sich im Isozentrum 9 schneiden. Der Halter 15 entfällt also bei diesem Ausführungsbeispiel. Dafür nimmt der Sockel 2a eine etwas größere Breite als der Sockel 2 ein.

Die Röntgenstrahler 6, 8 sind in einem gemeinsamen Gehäuse 20 in einer vertikalen Ebene verstellbar gelagert. Hierzu ist der Röntgenstrahler 6 in Richtung des Zentralstrahles 21 verstellbar, während der Röntgenstrahler 8 an einem Arm 22 befestigt ist, welcher um eine Achse 23 schwenkbar ist. In der Figur 5 ist dabei gestrichelt dargestellt, wie die Röntgenstrahler 6, 8 im Gehäuse 20 liegen, wenn sie nicht gebraucht werden. Das Gehäuse 20 ist mit einem Universalanschlußstück 24 versehen, das bei dem Beispiel gemäß den Figuren 5 und 6 an der Decke 25 des Behandlungsraumes befestigt ist.

Für die Ortung von Steinen nehmen die Strahler 6, 8 die in den Figuren 5 und 6 voll ausgezogen gezeichneten Stellungen ein. Werden sie nicht mehr benötigt, so werden sie in das Gehäuse 20 verstellt und stören deshalb bei der weiteren Behandlung, z.B. bei perkutanen Eingriffen (perkutane Nephrostomie), nicht, so daß ein freier Zugang zum Patienten gewährleistet ist.

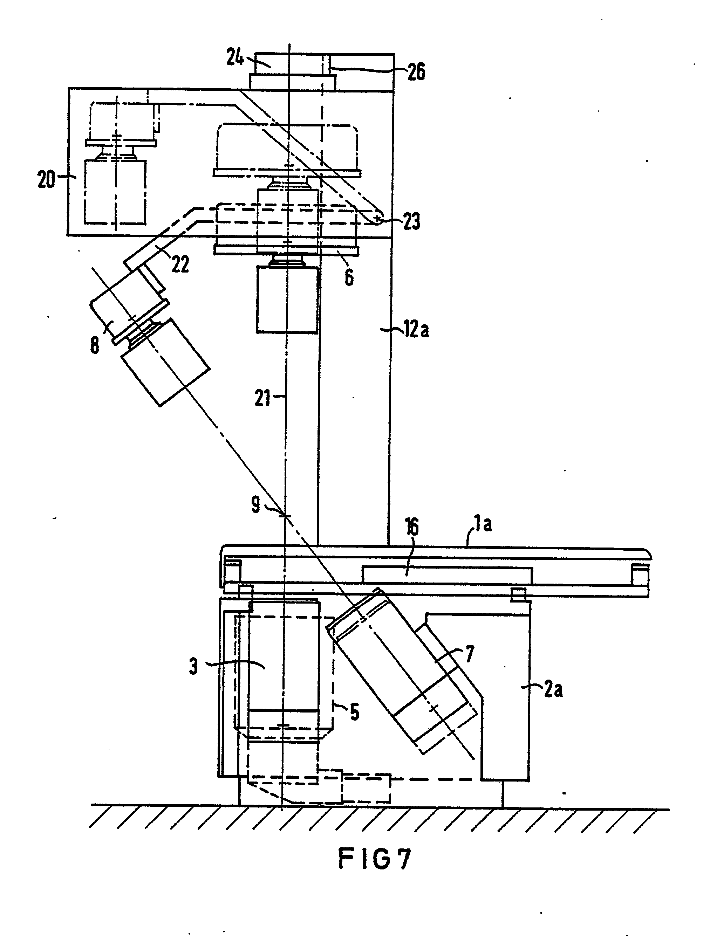

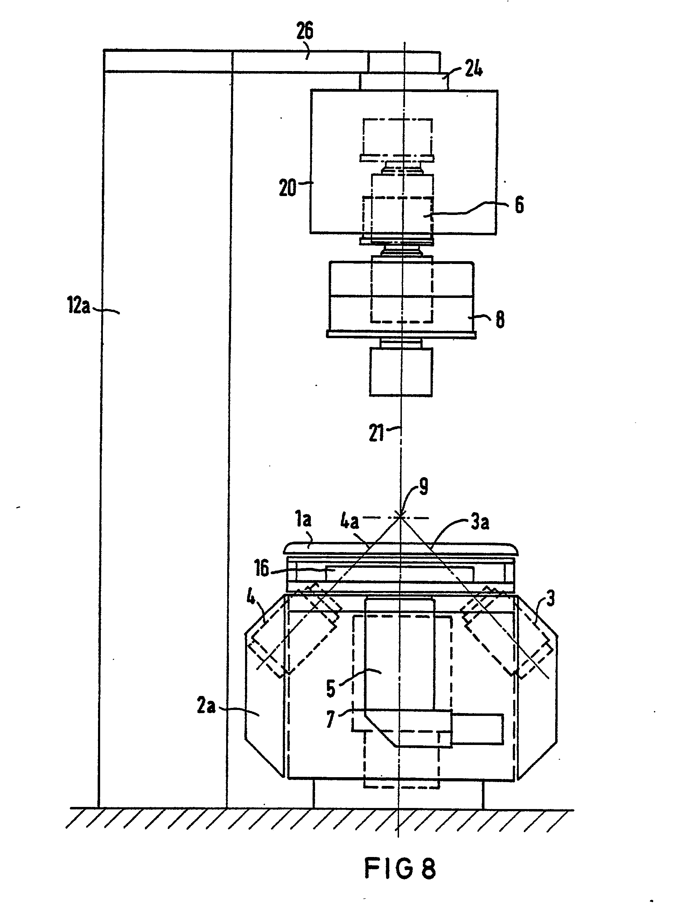

Die Figuren 7 und 8 zeigen, daß das Universalanschlußstück 24 auch an einem Arm 26 befestigt sein kann, welcher mit einer Säule 12a verbunden ist, die sich auf dem Fußboden des Behandlungsraumes abstützt. Der Arm 26 kann auch an der Wand des Untersuchungsraumes befestigt sein.

## Patentansprüche

1. Lithotripsie-Einheit, aufweisend folgende Merkmale:

a) einen in drei Raumkoordinaten in bezug auf ein Isozentrum (9) verstellbaren Patientenlagerumstich (1),

b) einen Stoßwellengenerator (3, 4) zur Zertrümmerung von im Körper eines Patienten befindlichen Steinen, wobei der Stoßwellengenerator (3, 4) über eine Membran an die Patientenoberfläche ankoppelbar ist und unter dem Patientenlagerungstisch (1) zumindest in Richtung seiner Achse (3a, 4a) derart einstellbar gelagert ist, daß eine Fokusverlagerung in das Isozentrum (9) möglich ist,

c) eine Röntgenuntersuchungseinrichtung zur Ortung zu zertrümmernder Steine, wobei die Röntgenuntersuchungseinrichtung eine Röntgeneinheit (5, 6; 7, 8) aufweist, mittels derer ein Patient unter unterschiedlichen Winkeln durchstrahlbar ist, wobei die durch die Zentralstrahlen (6a, 8a) der Röntgeneinheit (5, 6; 7, 8) definierte Ebene die Längsachse des Patientenlagerungstisches (1) enthält, derart, daß ein Zentralstrahl (6a) in anterior-posterior-Richtung und ein zweiter Zentralstrahl (8a) in caudal-cranialer Richtung verläuft und die Zentralstrahlen (6a, 8a) sich in dem Isozentrum (9) schneiden, und

d) der Patientenlagerungstisch (1) ist zum Anbau urologischer Hilfsgeräte und einer Röntgenaufnahmevorrichtung (16) ausgebildet.

2. Lithotripsie-Einheit nach Anspruch 1, dadurch gekennzeichnet, daß zwei Stoßwellengeneratoren (3, 4) auf einem gemeinsamen, um eine parallel zur Längsachse des Patientenlagerungstisches (1) verlaufende Achse (14) schwenkbaren Halter (15) derart gelagert sind, daß sich ihre Achsen (3a, 4a) unter einem Winkel schneiden und jeder Stoßwellengenerator (3, 4) in Richtung seiner Achse (3a, 4a) verstellbar ist.

3. Lithotripsie-Einheit nach Anspruch 1 oder 2, dadurch gekennzeichnet, daß der in anterior-posterior-Richtung strahlende Röntgenstrahler (6) der Röntgeneinheit (5, 6; 7, 8) quer zum Patientenlagerungstisch (1) verstellbar ist.

4. Lithotripsie-Einheit nach Anspruch 1 bis 3, dadurch gekennzeichnet, daß der in caudal-cranialer Richtung strahlende Röntgenstrahler (8) aus dem Arbeitsbereich herausbewegbar gelagert ist.

5. Lithotripsie-Einheit nach Anspruch 1, dadurch gekennzeichnet, daß beide Strahler (6, 8) in einem gemeinsamen Gehäuse (20) in einer vertikalen Ebene verstellbar gelagert sind.

6. Lithotripsie-Einheit nach Anspruch 5, dadurch gekennzeichnet, daß das Gehäuse (20) ein Universalanschlußstück (24) zum wahlweisen Verbinden mit der Decke (25) oder mit einem Arm (26) aufweist, welcher mit einer auf dem Fußboden abgestützten Säule (12a) oder der Wand des Untersuchungsraumes verbindbar ist.

**Claims**

1. Lithotripsy unit having the following features:
a) a patient support table (1) which is adjustable in three spatial directions in relation to an isocentre (9);
b) a shock wave generator (3, 4) for disintegrating calculi which are located in the body of a patient, in which case the shock wave generator (3, 4) can be coupled by way of a diaphragm to the surface of the patient and is mounted underneath the patient support table (1) so that it may be adjusted at least in the direction of its axis (3a, 4a) such that a displacement of the focus to the isocentre (9) is possible;
c) an X-ray examination device for locating calculi which are to be disintegrated, with the X-ray examination device having an X-ray unit (5, 6; 7, 8), by means of which it is possible to irradiate a patient at different angles, with the plane which is defined by the central rays (6a, 8a) of the X-ray unit (5, 6; 7, 8) including the longitudinal axis of the patient support table (1) such that a central ray (6a) runs in an anterior-posterior direction and a second central ray (8a) runs in a caudal-cranial direction and the central rays (6a, 8a) intersect in the isocentre (9) and
d) the patient support table (1) is suitable for the addition of urological auxiliary equipment and a radiograph arrangement (16).

2. Lithotripsy unit according to claim 1, characterised in that two shock wave generators (3, 4) are mounted on a common holder (15) which can be swung about an axis (14) which runs parallel to the longitudinal axis of the patient support table (1) such that their axes (3a, 4a) intersect at an angle and each shock wave generator (3, 4) is adjustable in the direction of its axis (3a, 4a).

3. Lithotripsy unit according to claim 1 or 2, characterised in that the X-ray source (6) of the X-ray unit (5, 6; 7, 8), which radiates in the anterior-posterior direction, is adjustable crosswise in relation to the patient support table (1).

4. Lithotripsy unit according to one of the claims 1 to 3, characterised in that the X-ray source (8), which radiates in the caudal-cranial direction, is mounted such that it can be moved out of the working area.

5. Lithotripsy unit according to claim 1, characterised in that both sources (6, 8) are mounted in a common housing (20) such that they are adjustable in a vertical plane.

6. Lithotripsy unit according to claim 5, characterised in that the housing (20) has a universal connection piece (24) for the selective connection with the ceiling (25) or with an arm (26) which is connectable with a column (12a) which is supported on the floor or with the wall of the examintion room.

**Revendications**

1. Unité pour la lithotritie, possédant les caractéristiques suivantes:
a) un plateau (1) formant couchette pour patient, déplaçable dans trois coordonnées de l'espace par rapport à un isocentre (9),
b) un générateur d'ondes de choc (3, 4) servant à fragmenter des calculs situés à l'intérieur du corps d'un patient, le générateur d'ondes de choc (3, 4) pouvant être couplé à la surface du patient par l'intermédiaire d'une membrane et étant monté au-dessous du plateau (1) formant couchette pour patient de manière à être réglable au moins dans la direction de son axe (3a, 4a), pour pouvoir déplacer le foyer à l'isocentre (9),
c) un appareil de radiodiagnostic servant à localiser des calculs devant être fragmentés, l'appareil de radiodiagnostic comportant une unité radiologique (5, 6; 7, 8) permettant d'irradier un patient sous des angles différents, le plan défini par les rayons centraux (6a, 8a) de l'unité radiologique (5, 6; 7, 8) contenant l'axe longitudinal du plateau (1) formant couchette pour patient de sorte qu'un rayon central (6a) s'étend dans la direction antéro-postérieure, qu'un second rayon central (8a) s'étend dans la direction de la partie candal-cranienne et que les rayons centraux (6a, 8a) se coupent à l'isocentre (9), et
d) le plateau (1) formant couchette pour patient est agencé pour permettre le montage d'appareils auxiliaires d'urologie et d'un dispositif de radiographie (16).

2. Unité pour la lithotritie suivant la revendication 1, caractérisé par le fait que deux générateurs d'ondes de choc (3, 4) sont montés sur un support commun (15), pouvant pivoter autour d'un axe (14) parallèle à l'axe longitudinal du plateau (1) formant couchette pour patient (1), de sorte que leurs axes (3a, 4a) se coupent sous un certain angle et que chaque générateur d'ondes de choc (3, 4) est déplaçable dans la direction de son axe (3a, 4a).

3. Unité pour la lithotritie suivant la revendication 1 ou 2, caractérisée par le fait que l'émetteur (6) de rayons X, qui émet dans la direction antéro-postérieure, de l'unité radiologique (5, 6; 7, 8) est déplaçable transversalement par rapport au plateau (1) formant couchette pour patient (1).

4. Unité pour la lithotritie suivant l'une des revendications 1 à 3, caractérisée par le fait que l'émetteur (8) de rayons X qui émet dans la direction candal-cranienne, est monté de manière à pouvoir être amené hors de la zone de travail.

5. Unité pour la lithotritie suivant la revendication 1, caractérisée par le fait que les deux émetteurs (6, 8) sont montés dans un boîtier commun (20) de manière à être déplaçables dans un plan vertical.

6. Unité pour la lithotritie suivant la revendication 5, caractérisée par le fait que le boîtier (20) possède un raccord universel (24) permettant de la relier, au choix, au plafond (25) ou à un bras (26), qui peut être relié à une colonne (12a) en appui au sol ou au mur de la salle d'examen.

FIG 1

FIG 2

FIG 3

11

13

6

8

10

12

1

4a 5; 7 3a

4 3

14

2 15

FIG 4

FIG 5

FIG 6

FIG 7

FIG 8